# EUROPEAN PATENT APPLICATION

(11) **EP 1 281 960 A2**
(43) Date of publication of application: **05.02.2003**
(21) Application number: 02255254.1
(22) Date of filing: 26.07.2002
(51) Int. Cl.: G01N 33/48

(54) **Methods and devices for use in analyte concentration determination assays**

(30) Priority: 01.08.2001 US 920263
(71) Applicant: Lifescan, Inc., Milpitas, CA 95035 (US)
(72) Inventor: Teodorcyzk, Maria, San Jose, California 95135 (US); Shar, Mahesh, Santa Clara, California 95051 (US); O'Hara, Timothy James, San Ramon, California 94583 (US)
(74) Representative: Mercer, Christopher Paul

(57) **Abstract**

Methods and devices are provided for use in the determination of the concentration of an analyte in a sample. In the subject methods, a sample is introduced to a reagent test strip, where the sample is either a test fluid or a control fluid, where the control fluid is free of a mediator dissolution slowing component and an oxidizing agent when used with an electrochemical analyte concentration determination assay. The concentration of analyte in the sample is determined and the sample is identified as a control fluid or a test fluid. Also provided are devices for determining the concentration of an analyte in a sample, where the devices have a sample identification element for identifying whether a sample is a control or a test fluid. The subject methods and devices find use in a variety of different applications, particularly in the determination of blood glucose concentrations.

## Description

### FIELD OF THE INVENTION

The field of this invention is analyte concentration determination, particularly blood analyte concentration determination and more particularly blood glucose concentration determination.

### BACKGROUND OF THE INVENTION

Analyte concentration determination in physiological fluids, *e.g.,* blood or blood derived products such as plasma, is of ever increasing importance to today's society. Such assays find use in a variety of applications and settings, including clinical laboratory testing, home testing, etc., where the results of such testing play a prominent role in the diagnosis and management of a variety of disease conditions. Analytes of interest include glucose for diabetes management, cholesterol for monitoring cardiovascular conditions, and the like. In response to this growing importance of analyte concentration detection, a variety of analyte detection protocols and devices for both clinical and home use have been developed. Two common protocols that have been developed employ colorimetric methods and electrochemical methods.

Colorimetric-based analyte concentration determination assays are often based on the production of hydrogen peroxide and the subsequent detection thereof. Analyte concentrations that may be determined using such assays include: cholesterol, triglycerides, glucose, ethanol and lactic acid. For example, glucose is quantitated using such assays by first oxidizing glucose with glucose oxidase to produce gluconic acid and hydrogen peroxide. The resultant hydrogen peroxide, in conjunction with a peroxidase, causes the conversion of one or more organic substrates, *i.e.,* an indicator, into a chromogenic product, which product is then detected and related to the glucose concentration in the initial sample.

The other common analyte concentration determination assays use electrochemical-based methods. In such methods, an aqueous liquid sample is placed into a reaction zone in an electrochemical cell made up of at least two electrodes, *i.e.,* a reference and working electrode, where the electrodes have an impedance which renders them suitable for amperometric measurement. The component to be analyzed is allowed to react directly with an electrode, or directly or indirectly with a redox reagent to form an oxidisable (or reducible) substance in an amount corresponding to the concentration of the component to be analyzed, *i.e*., analyte. The quantity of the oxidisable (or reducible) substance present is then estimated electrochemically and related to the amount of analyte present in the initial sample.

Regardless of which type of analyte concentration determination system is used, an automated device, *e.g.*, an electrochemical meter or an optical meter depending on the type of assay, is typically employed for determining the concentration of the analyte in the sample. Many meters advantageously allow for an analyte concentration, and usually a plurality of analyte concentrations, to be stored in the memory of the meter. This feature provides the user with the ability to review analyte concentration levels over a period of time, often times as an average of previously collected analyte levels, where such averaging is performed according to an algorithm associated with the meter. However, to ensure that the meter is functioning properly, the user will usually introduce a control fluid to the system to test the meter before a test fluid is introduced, where the control fluid includes a known level of the analyte of interest. As such, analyte concentration levels of control fluid are stored in the memory of the meter, along with test fluid analyte levels. Thus, when a user seeks to review an average of test fluid analyte levels from previous tests, the results are skewed because of the inclusion of control fluid analyte levels into the averaging formula.

As such, it is desirable to be able to "flag' or identify control and test fluids as such. Flagging the fluids as either control or test fluids may be done manually, however it is desirable to do so automatically, which minimizes user interaction, thereby increasing ease-of-use.

One technique has been developed which uses a control solution formulated so that a device recognizes it as something other than blood (see U.S. Patent No. 5,723,284). However, in this protocol, the device automatically excludes the results of the solution from its memory, thereby preventing the storage of the control solution results if desirable. Furthermore, this protocol is limited for use only with an electrochemical-type method of analyte determination and necessarily requires a particular type of control fluid that includes specific reagents, thereby adding to the complexities and cost of the system.

As such, there is continued interest in the development of new methods and devices for use in the determination of analyte concentrations in a sample. Of particular interest would be the development of such methods and devices that include the ability to automatically flag a sample as control fluid or test fluid and to store or exclude measurements accordingly. Of particular interest would be the development of such methods that are suitable for use with electrochemical and colorimetric based analyte concentration determination assays.

### Relevant Literature

U.S. Patent documents of interest include 4,224,125; 4,545,382; 5,834,224; 5,942,102; 5,972,199 and 5,972,294.

### SUMMARY OF THE INVENTION

Methods and devices are provided for use in the determination of the concentration of an analyte in a sample. In the subject methods, a sample is introduced to a reagent test strip, where the sample is either a test fluid or a control fluid, where the control fluid is free of a mediator dissolution slowing component and an oxidizing agent when used with an electrochemical analyte concentration determination assay. The concentration of analyte in the sample is determined and the sample is identified as a control fluid or a test fluid. Also provided are devices for determining the concentration of an analyte in a sample, where the devices have a sample identification element for identifying whether a sample is a control or a test fluid. The subject methods and devices find use in a variety of different applications, particularly in the determination of blood glucose concentrations.

### BRIEF DESCRIPTION OF THE FIGURES

Figure 1 is a graph of data generated from an electrochemical analyte concentration determination assay used to determine analyte concentration values of a control fluid and a test fluid.

Figure 2 is a graph of data generated from a colorimetric analyte concentration determination assay used to determine analyte concentration values of a control fluid and a test fluid.

### DETAILED DESCRIPTION OF THE INVENTION

Methods and devices are provided for use in the determination of the concentration of an analyte in a sample. In the subject methods, a sample is introduced to a reagent test strip, where the sample is either a test fluid or a control fluid, where the control fluid is free of a mediator dissolution slowing component and an oxidizing agent when used with an electrochemical analyte concentration determination assay. The concentration of analyte in the sample is determined and the sample is identified as a control fluid or a test fluid. Also provided are devices for determining the concentration of an analyte in a sample, where the devices have a sample identification element for identifying whether a sample is a control or a test fluid. The subject methods and devices find use in a variety of different applications, particularly in the determination of blood glucose concentrations. In further describing the subject invention, the subject methods will be described first, followed by a review of the subject devices for use in practicing the subject methods.

Before the present invention is described, it is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an", and "the" include plural referents unless the context clearly dictates otherwise. Thus, for example, reference to "a test strip" includes a plurality of such test strips and reference to "the processor" includes reference to one or more processors and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### METHODS

As summarized above, the subject invention provides methods for determining the concentration of an analyte in a sample, where the methods advantageously allow for the determination of whether the sample is a control or a test fluid. The subject methods find use in the determination of a variety of different analyte concentrations, where representative analytes include glucose, cholesterol, lactate, alcohol, and the like. In many embodiments, the subject methods are employed to determine the glucose concentration in a test fluid, *e.g.*, a physiological sample.

While in principle the subject methods may be used to determine the concentration of an analyte in a variety of different physiological samples, such as urine, tears, saliva, and the like, they are particularly suited for use in determining the concentration of an analyte in blood or blood fractions, and more particularly in whole blood.

Similarly, a variety of different control fluids may be suitable for use with the subject invention depending on the type of assay system employed and the analyte of interest. However, generally, the control fluid is an aqueous solution that includes a predetermined amount of the analyte of interest, where the amount of analyte will necessarily vary. Typically, the amount of analyte ranges from about 20 mg/dL to 600 mg/dL, usually from about 40 mg/dL to 450 mg/dL. Other components of the control fluid may include one or more of the following: buffer (e.g., phosphate, citrate, citraconate), surfactant (e.g., FC 171, Triton X-100, Pluronic 25R2 manufactured by BASF Corp.), dispersant (e.g., Aerosil 200 manufactured by Degussa Corp.), polymer (e.g., methylcellulose, carboxymethyl cellulose, dextran, or polyvinyl acetate), dye (e.g., copper phthalocyanine-3,4',4",4"' - tetrasulfonic acid, tetrasodium salt; 1-[(6-methoxy-4-sulfo-m-tolyl)azo]-2-naphthol-6-sulfonic acid disodium salt), antifoaming agents (e.g., Dow B emulsion, manufactured by Dow Coming Corp.) and preservative (e.g., sodium benzoate, methyl paraben, EDTA, Germal II manufactured by Sutton Laboratories).

In certain embodiments, for example when employing electrochemical analyte concentration determination methods, the control fluid is generally one that it is free of mediator dissolution slowing components such as ethylene glycol, N-methylypyrrolidone and N-propanol and oxidizing agents such as potassium permanganate, potassium perchromate, potassium dichromate, potassium ferricyanide, sodium perchlorate and sodium periodate. In many embodiments, the control fluid is substantially free of any redox constituents, particularly when the control fluid is for use with electrochemical assays. In still other embodiments, the control fluid may include a reflectance component that is capable of generating a reflectance profile different from one generated by blood. For example, a suitable dye may be included, in other words a dye that is a modifier of reflectance and absorbance wherein the dye has a maximum absorbance of visual light outside that of hemoglobin, particularly when the control fluid is one which is prepared to be suitable for use with colorimetric assays. Representative dyes suitable for use in control fluids used with colorimetric assays and methods include, but are not limited to, copper phthalocyanine-3,4',4",4"' - tetrasulfonic acid, tetrasodium salt, 3,7-bis(dimethylamino)phenothiazin-5-ium chloride, copper(II) phthalocyanine and 1-(1-naphthylazo)-2-naphthol-3,6-disulfonic acid disodium salt.

In practicing the subject methods, the first step in the subject methods is to provide a reagent test strip having a sample receiving region and introduce a sample into the sample receiving area, *i.e*., the reaction area of the test strip. The sample may be introduced into the reaction area using any convenient protocol, where the sample may be injected into the reaction area, allowed to wick into the reaction area, and the like, as may be convenient. Various reagent test strips may be used with the subject inventions, depending on the type of analyte concentration determination assay used. Two types of analyte concentration determination assays suitable for use with the subject methods are electrochemical analyte concentration determination systems and colorimetric analyte concentration determination systems, where each of these will be described below to provide a proper foundation for the invention.

### Electrochemical Analyte Concentration Determination Systems and Methods of Use

The first step in the subject methods employing an electrochemical assay system is to introduce a quantity of the sample of interest, *e.g.,* a physiological sample, into an electrochemical cell, *e.g.*, by introducing the sample into a sample receiving area of an electrochemical test strip, etc. The physiological sample may vary, but in many embodiments is generally whole blood or a derivative or fraction thereof, where whole blood is of particular interest in many embodiments. The amount of physiological sample, *e.g*. blood, that is introduced into the reaction area of the test strip varies, but generally ranges from about 0.1 to 10 µL, usually from about 0.3 to 1.6 µL. The sample is introduced into the reaction area using any convenient protocol, where the sample may be injected into the reaction area, allowed to wick into the reaction area, and the like, as may be convenient.

While the subject methods may be used, in principle, with any type of electrochemical cell having spaced apart working and reference electrodes, as described above, in many embodiments the subject methods employ an electrochemical test strip. The electrochemical test strips employed in these embodiments of the subject invention are made up of two opposing metal electrodes separated by a thin spacer layer, with a cut out section that defines a reaction area or zone. In many embodiments a redox reagent system is located in the reaction area or zone.

In certain embodiments of these electrochemical test strips, the working and reference electrodes are generally configured in the form of elongated rectangular strips. Typically, the length of the electrodes ranges from about 1.9 to 4.5 cm, usually from about 2.0 to 2.8 cm. The width of the electrodes ranges from about 0.07 to 0.76 cm, usually from about 0.24 to 0.60 cm. The working and reference electrodes typically have a thickness ranging from about 10 to 100 nm and usually from about 10 to 60 nm.

The working and reference electrodes are further characterized in that at least the surface of the electrodes that faces the reaction area of the electrochemical cell in the strip is a metal, where metals of interest include palladium, gold, platinum, silver, iridium, carbon (conductive carbon ink), doped tin oxide, stainless steel and the like. In many embodiments, the metal is gold or palladium. While in principle the entire electrode may be made of the metal, each of the electrodes is generally made up of an inert support material on the surface of which is present a thin layer of the metal component of the electrode. In these more common embodiments, the thickness of the inert backing material typically ranges from about 25 to 500, usually 50 to 400 µm, *e.g.,* from about 127 to 178 µm, while the thickness of the metal layer typically ranges from about 10 to 100 nm and usually from about 10 to 60 nm, *e.g*. a sputtered metal layer. Any convenient inert backing material may be employed in the subject electrodes, where typically the material is a rigid material that is capable of providing structural support to the electrode and, in turn, the electrochemical test strip as a whole. Suitable materials that may be employed as the backing substrate include plastics, *e.g.*, PET, PETG, polyimide, polycarbonate, polystyrene, silicon, ceramic, glass, and the like.

A feature of the electrochemical test strips used in these embodiments of the subject methods is that the working and reference electrodes as described above generally face each other and are separated by only a short distance, such that the distance between the working and reference electrodes in the reaction zone or area of the electrochemical test strip is extremely small. This minimal spacing of the working and reference electrodes in the subject test strips is a result of the presence of a thin spacer layer positioned or sandwiched between the working and reference electrodes. The thickness of this spacer layer may range from 50 to 750 µm and is often less than or equal to 500 µm, and usually ranges from about 100 to 175 µm, *e.g*., 102 to 153 µm. The spacer layer is cut so as to provide a reaction zone or area with at least an inlet port into the reaction zone, and generally an outlet port out of the reaction zone as well. The spacer layer may have a circular reaction area cut with side inlet and outlet vents or ports, or other configurations, *e.g.* square, triangular, rectangular, irregular shaped reaction areas, etc. The spacer layer may be fabricated from any convenient material, where representative suitable materials include PET, PETG, polyimide, polycarbonate, and the like, where the surfaces of the spacer layer may be treated so as to be adhesive with respect to their respective electrodes and thereby maintain the structure of the electrochemical test strip.

The electrochemical test strips used in these embodiments of the subject invention include a reaction zone, area or sample receiving region that is defined by the working electrode, the reference electrode and the spacer layer, where these elements are described above. Specifically, the working and reference electrodes define the top and bottom of the reaction area, while the spacer layer defines the walls of the reaction area. The volume of the reaction area typically ranges from about 0.1 to 10 µL, usually from about 0.2 to 5.0 µL, and more usually from about 0.3 to 1.6 µL.

In many embodiments, a reagent system or composition is present in the reaction area, where the reagent system interacts with components in the fluid sample during the assay. Reagent systems of interest typically include a redox couple.

The redox couple of the reagent composition, when present, is made up of one or more redox couple agents. A variety of different redox couple agents are known in the art and include: ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, ruthenium complexes, and the like. In many embodiments, redox couples of particular interest are ferricyanide, and the like.

Other reagents that may be present in the reaction area include buffering agents, *e.g*. citraconate, citrate, malic, maleic, phosphate, "Good" buffers and the like. Yet other agents that may be present include: divalent cations such as calcium chloride, and magnesium chloride; surfactants such as Triton, Macol, Tetronic, Silwet, Zonyl, and Pluronic; stabilizing agents such as albumin, sucrose, trehalose, mannitol, and lactose.

Examples of such a reagent test strips suitable for use with the subject invention include those described in EP-A-1 067 384, WO 01/57510 and WO 01/57238.

Generally for electrochemical assays, an electrochemical measurement is made using the reference and working electrodes. The electrochemical measurement that is made may vary depending on the particular nature of the assay and the device with which the electrochemical test strip is employed, *e.g*., depending on whether the assay is coulometric, amperometric or potentiometric. Generally, the electrochemical measurement will measure charge (coulometric), current (amperometric) or potential (potentiometric), usually over a given period of time following sample introduction into the reaction area. Methods for making the above described electrochemical measurement are further described in U.S. patent Nos.: 4,224,125; 4,545,382; and 5,266,179; as well as WO 97/18465; WO 99/49307; the disclosures of the priority documents of which are herein incorporated by reference.

Following detection of the electrochemical signal generated in the reaction zone as described above, the amount of the analyte present in the sample introduced into the reaction zone is then determined by relating the electrochemical signal to the amount of analyte in the sample. Representative meters for automatically practicing these steps are, further described in EP-A-1 067 384, WO 01/57510 and WO 01/57238.

### Colorimetric Analyte Concentration Determination Systems and Methods of Use

Similar to the electrochemical systems described above, the first step in the subject methods employing a colorimetric assay system is to introduce a quantity of the sample of interest, *e.g*., a physiological sample, to a reagent test strip, and more specifically to a sample receiving area of a colorimetric test strip, where the terms colorimetric and photometric are herein used interchangeably. The physiological sample may vary, but in many embodiments is generally whole blood or a derivative or fraction thereof, where whole blood is of particular interest in many embodiments.

The colorimetric reagent test strips employed in these embodiments of the subject invention are generally made up of at least the following components: a porous matrix for receiving a sample and a reagent composition that typically includes one or more members of an analyte oxidation signal producing system.

The matrix that is employed in the subject test strips is an inert porous matrix which provides a support for the various members of the signal producing system, described infra, as well as the light absorbing or chromogenic product produced by the signal producing system, *i.e.,* the indicator. The inert porous matrix is configured to provide a location for the physiological sample, *e.g.,* blood, application and a location for the detection of the light-absorbing product produced by the indicator of the signal producing system. As such, the inert porous matrix is one that is permissive of aqueous fluid flow through it and provides sufficient void space for the chemical reactions of the signal producing system to take place. A number of different porous matrices have been developed for use in various analyte detection assays, which matrices may differ in terms of materials, pore sizes, dimensions and the like, where representative matrices include those described in U.S. Patent Nos.: 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference. In principle, the nature of the porous matrix is not critical to the subject test strips and therefore is chosen with respect to the other factors, including the nature of the instrument which is used to read the test strip, convenience and the like. As such, the dimensions and porosity of the test strip may vary greatly, where the matrix may or may not have a porosity gradient, *e.g.* with larger pores near or at the sample application region and smaller pores at the detection region. Materials from which the matrix may be fabricated vary, and include polymers, *e.g.* polysulfone, polyamides, cellulose or absorbent paper, and the like, where the material may or may not be functionalized to provide for covalent or non-covalent attachment of the various members of the signal producing system.

In addition to the porous matrix, the subject test strips further include one or more members of a signal producing system which produces a detectable product in response to the presence of analyte, which detectable product can be used to derive the amount of analyte present in the assayed sample. In the subject test strips, the one or more members of the signal producing system are associated, *e.g.* covalently or non-covalently attached to, at least a portion of (*i.e*., the detection region) the porous matrix, and in many embodiments to substantially all of the porous matrix.

The signal producing system is an analyte oxidation signal producing system. By analyte oxidation signal producing system is meant that in generating the detectable signal from which the analyte concentration in the sample is derived, the analyte is oxidized by a suitable enzyme to produce an oxidized form of the analyte and a corresponding or proportional amount of hydrogen peroxide. The hydrogen peroxide is then employed, in turn, to generate the detectable product from one or more indicator compounds, where the amount of detectable product generated by the signal measuring system, *i.e.* the signal, is then related to the amount of analyte in the initial sample. As such, the analyte oxidation signal producing systems present in the subject test strips are also correctly characterized as hydrogen peroxide based signal producing systems.

As indicated above, the hydrogen peroxide based signal producing systems include an enzyme that oxidizes the analyte and produces a corresponding amount of hydrogen peroxide, where by corresponding amount is meant that the amount of hydrogen peroxide that is produced is proportional to the amount of analyte present in the sample. The specific nature of this first enzyme necessarily depends on the nature of the analyte being assayed but is generally an oxidase. As such, the first enzyme may be: glucose oxidase (where the analyte is glucose); cholesterol oxidase (where the analyte is cholesterol); alcohol oxidase (where the analyte is alcohol); lactate oxidase (where the analyte is lactate) and the like. Other oxidizing enzymes for use with these and other analytes of interest are known to those of skill in the art and may also be employed. In those preferred embodiments where the reagent test strip is designed for the detection of glucose concentration, the first enzyme is glucose oxidase. The glucose oxidase may be obtained from any convenient source, *e.g*. a naturally occurring source such as Aspergillus niger or Penicillum, or recombinantly produced.

The second enzyme of the signal producing system is an enzyme that catalyzes the conversion of one or more indicator compounds into a detectable product in the presence of hydrogen peroxide, where the amount of detectable product that is produced by this reaction is proportional to the amount of hydrogen peroxide that is present. This second enzyme is generally a peroxidase, where suitable peroxidases include: horseradish peroxidase (HRP), soy peroxidase, recombinantly produced peroxidase and synthetic analogs having peroxidative activity and the like. See *e.g.,* Y. Ci, F. Wang; Analytica Chimica Acta, 233 (1990), 299-302.

The indicator compound or compounds, *e.g*. substrates, are ones that are either formed or decomposed by the hydrogen peroxide in the presence of the peroxidase to produce an indicator dye that absorbs light in a predetermined wavelength range. Preferably the indicator dye absorbs strongly at a wavelength different from that at which the sample or the testing reagent absorbs strongly. The oxidized form of the indicator may be the colored, faintly-colored, or colorless final product that evidences a change in color of the testing side of the membrane. That is to say, the testing reagent can indicate the presence of glucose in a sample by a colored area being bleached or, alternatively, by a colorless area developing color.

Indicator compounds that are useful in the present invention include both one- and two-component chromogenic substrates. One-component systems include aromatic amines, aromatic alcohols, azines, and benzidines, such as tetramethyl benzidine-HCl. Suitable two-component systems include those in which one component is MBTH, an MBTH derivative (see for example those disclosed in U.S. Patent Application Ser. No. 08/302,575, incorporated herein by reference), or 4-aminoantipyrine and the other component is an aromatic amine, aromatic alcohol, conjugated amine, conjugated alcohol or aromatic or aliphatic aldehyde. Exemplary two-component systems are 3-methyl-2-benzothiazolinone hydrazone hydrochloride (MBTH) combined with 3-dimethylaminobenzoic acid (DMAB); MBTH combined with 3,5-dichloro-2-hydroxybenzene-sulfonic acid (DCHBS); and 3-methyl-2-benzothiazolinonehydrazone N-sulfonyl benzenesulfonate monosodium (MBTHSB) combined with 8-anilino-1 naphthalene sulfonic acid ammonium (ANS). In certain embodiments, the dye couple MBTHSB-ANS is preferred.

In yet other embodiments, signal producing systems that produce a fluorescent detectable product (or detectable non- fluorescent substance, *e.g.* in a fluorescent background) may be employed, such as those described in: Kiyoshi Zaitsu, Yosuke Ohkura: New fluorogenic substrates for Horseradish Peroxidase: rapid and sensitive assay for hydrogen peroxide and the Peroxidase. Analytical Biochemistry (1980) 109, 109-113.

Generally, for colorimetric assays, the sample is allowed to react with the members of the signal producing system to produce a detectable product that is present in an amount proportional to the initial amount present in the sample. The amount of detectable product, *i.e*., signal produced by the signal producing system, is then determined and related to the amount of analyte in the initial sample. As described, in certain embodiments, automated meters, *i.e.,* optical meters, that perform the above mentioned detection and relation steps are employed. The above described reaction, detection and relating steps, as well as instruments for performing the same, are further described in U.S. Patent Nos. 4,734,360; 4,900,666; 4,935,346; 5,059,394; 5,304,468; 5,306,623; 5,418,142; 5,426,032; 5,515,170; 5,526,120; 5,563,042; 5,620,863; 5,753,429; 5,573,452; 5,780,304; 5,789,255; 5,843,691; 5,846,486; 5,968,836 and 5,972,294; the disclosures of which are herein incorporated by reference.

Examples of such colorimetric or photometric reagent test strips suitable for use with the subject invention include those described in U.S. Patent Nos.: 5,563,042; 5,753,452; 5,789,255, herein incorporated by reference.

### Flagging of Control and Test Fluids

As summarized above, a feature of the subject methods is that the sample is flagged or determined to be either a control fluid or a test fluid. In other words, the value or signal generated by the signal producing system of a colorimetric reagent test strip or value or the electrochemical signal generated in the reaction zone of an electrochemical reagent test strip is obtained by the meter and, in addition to being used for analyte concentration determination, is employed for fluid identification as either a test or a control fluid.

The identification of the fluid as a test fluid or a control fluid can be made either before or after, or even during, the analyte concentration determination step. As such, the identification can be made either simultaneously with, or sequentially with respect to, analyte concentration determination.

Regardless of when sample identification occurs in relation to the analyte determination step, usually sample identification occurs within as short period of time. By short period of time is meant less than about 20 seconds. In embodiments employing electrochemical methods, sample identification usually occurs in less than about 1 second from the time of sample introduction and more usually occurs in about 0.25-0.75 seconds from the time of sample introduction. In embodiments employing colorimetric methods, sample identification usually occurs in about 10 -20 seconds from sample introduction and more usually in about 12-18 seconds from the time of sample introduction. As such, analyte concentration determination may take less time, the same amount of time or a greater amount of time than sample identification, depending on the particular system and analyte of interest, for example analyte concentration determination may take longer than 0.25-1 second, and may take longer than 1-20 seconds.

Generally, fluid or sample identification, *i.e.,* identification of a fluid as either a control or a test fluid, involves obtaining a signal or measurement and comparing it to a reference value in order to derive or determine the identity of the fluid. By reference is meant a predetermined or standard signal, concentration level, or the like which is known, *e.g*., a standard signal level known to the user or programmed into a meter, to which the sample, *e.g*., the signal associated with the sample, is compared. The reference values are typically ones that are observed in less than about 20 seconds from the time of sample introduction to the meter. For example, the reference value may be a reflectance value or a current value, where the current value is one that is typically observed in less than about 1 second from the time said sample is introduced to the meter and usually observed in about 0.25 to 0.75 seconds from sample introduction. The reflectance value is one that is typically observed in about 10-20 from sample introduction and usually about 12-18 seconds from sample introduction to the meter. In obtaining the requisite signals or measurements from the sample, *e.g.,* reflectance values or electrochemical measurements, the signals may be obtained periodically or substantially continuously, if not continuously, during the short period of time.

As described above, once the signal or measurement has been obtained from the sample, the identity of the sample is then determined based upon the obtained signal's relation to a reference value , *e.g.,* a standard or predetermined value or level. For example, during the short period of time in which the identity of the sample is determined, the signal generated from the application of the sample to the reagent test strip is compared to a predetermined reference value, *e.g.,* a current flow value, reflectance value, or the like, which may be stored in a meter and to which the sample signal is compared. As such, the identity of a sample is accomplished by distinguishing between the signals of the two fluids due to the difference in signals generated by each of the fluids and comparing that signal to a known signal level or reference. For example, in certain embodiments, the identity of a sample may be determined by obtaining and comparing the signal generated by the sample to a known reference, whereby the signal level is found to be lower than the reference and therefore the sample may be flagged and identified as a control fluid. Alternatively, the signal level may be found to be equal to or significantly higher than the reference and the sample may be flagged and identified as test fluid, or *vice versa.* More specifically, in certain embodiments employing electrochemical methods, a sample may be identified as a control fluid if it is above a reference value, *e.g*., a reference current value, and identified as a test fluid if it is below such a reference value. In certain embodiments employing colorimetric methods, a sample may be identified as a control fluid if it is below a reference value, e.g., a reference reflectance value such as a K/S value as will be described in more detail below, and identified as a test fluid if it is above such a reference value.

Regardless of how the identity of the sample is compared to a known signal level, once the analyte concentration of the sample has been identified and an identification has been made as to whether the sample is a control or a test fluid, the identified sample or analyte concentration thereof may be further processed accordingly. For example, an identified sample may then be stored or excluded from the memory of the meter where such storing or excluding may be accomplished manually by the user or automatically by the meter. For example, in many embodiments, a signal or measurement, *e.g.,* a concentration level of an analyte, identified as that from a control fluid sample may be excluded from the memory of the meter while a signal or concentration level identified as that from test fluid may be stored. Alternatively, a signal or concentration level which has been flagged or identified as that from a control fluid may be stored in the meter, *e.g.,* in a memory element of a meter, where it is distinguishable from stored signals or concentration levels flagged as those from test fluid.

Distinguishing signals as either control or test signals may be accomplished by a variety of methods, for example, a control fluid signal may be stored in a separate portion of the memory or may be stored along with test fluid readings, where it remains distinguished from test readings also stored, *e.g.,* a control and/or a test fluid is "marked", "flagged", or otherwise distinguishable as a control or a test fluid. Regardless of whether the control fluid reading is stored in a separate memory portion or not, control or test fluid analyte concentrations stored in the memory of a meter may be reviewed and/or monitored separately, such that analyte levels, and in particular an average of a plurality of analyte levels of each may be viewed by a user without being "skewed' by the inclusion of analyte concentrations from a different source. For example, a plurality of analyte concentrations may be stored in a memory element of a meter such that an average of a plurality of measurements, *e.g.,* 7, 14 and 30 days of test fluid analyte concentrations, may be reviewed without the inclusion of control fluid concentration values, *e.g.,* blood glucose concentrations may be stored and averaged so that the user may review and monitor average glucose levels obtained from test fluid over time without having such averages of test fluid include control fluid values. As described above, a meter may perform the aforementioned storage/exclusion functions automatically.

### SYSTEMS

The above described methods find use with systems that are made up of test strips and automated devices, i.e., meters, for reading these test strips. Each of these systems is now described in greater detail.

### DEVICES

Also provided by the subject invention are meters for practicing the subject invention, generally electrochemical and colorimetric, *i.e.,* optical or photometric, automated meters. Although the meters are suitable for use with a wide variety of analytes, they are particularly well suited for use in the determination of glucose, and in particular glucose in whole blood. Automated meters for use with electrochemical and colorimetric assays in the determination of analyte concentrations in samples are well known in the art, for example see U.S. Patent No. 5,059,394 and copending U.S. Application Serial No. 09/333,793, the disclosures of which are herein incorporated by reference. Regardless of the type of assay system or meter used, a common feature of the meter is that it includes a memory element or system for storing data and a microprocessor or other programmed electronic control and display circuits adapted to perform the required operations and calculations and to display the results. For example, microprocessors associated with analyte concentration determination meters may serve to control functions including timing for the entire system, and together with program and data memory system or element, storing data corresponding to analyte concentration levels.

A feature of the meter of the present invention is that it has a sample identification element that is capable of identifying whether a sample is a test fluid or a control fluid. A variety of different control fluids may be suitable for use with the subject invention depending on the type of assay system employed and the analyte of interest. However, generally, the control fluid is an aqueous solution that includes a predetermined amount of the analyte of interest, where the amount of analyte will necessarily vary. Typically, the amount of analyte ranges from about 20 mg/dL to 600 mg/dL, usually from about 40 mg/dL to 450 mg/dL. Other components of the control fluid may include one or more of buffers, surfactants, dispersants, polymers, dyes, preservatives and antifoaming agents.

For the control fluids used with electrochemical analyte concentration determination assays and methods, the control fluid is generally one that it is free of mediator dissolution slowing components such as ethylene glycol, N-methylypyrrolidone and N-propanol and oxidizing agents such as potassium permanganate, potassium perchromate, potassium dichromate, potassium ferricyanide, sodium perchlorate and sodium periodate. In many embodiments, the control fluid is substantially free of any redox constituents, particularly when the control fluid is for use with electrochemical assays. In still other embodiments, the control fluid may include a reflectance component that is capable of generating a reflectance profile different from one generated by blood. For example, a suitable dye may be included, in other words a dye that is a modifier of reflectance and absorbance wherein the dye has a maximum absorbance of visual light outside that of hemoglobin, particularly when the control fluid is one which is prepared to be suitable for use with colorimetric assays. Representative dyes suitable for use in control fluids used with colorimetric assays and methods include, but are not limited to, copper phthalocyanine-3,4',4",4"' - tetrasulfonic acid, tetrasodium salt, 3,7-bis(dimethylamino)phenothiazin-5-ium chloride, copper(II) phthalocyanine and 1-(1-naphthylazo)-2-naphthol-3,6-disulfonic acid disodium salt.

The sample identification element, *i.e.,* a microprocessor, may be programmed to automatically identify a sample according to its respective signal values, or the like. By signal is meant one or more data points generated by a sample, oftentimes a set of data points over a period of time. In obtaining the requisite signals or measurements from the sample, *e.g.,* signals related to the reflectance of light generated by a colorimetric reaction or related to the current generated by an electrochemical reaction, the signals may be obtained periodically or substantially continuously, if not continuously, during a short period of time, usually less than about 20 seconds.

Accordingly, the meter also includes one or more reference values to which one or more signals generated by the sample is compared for identifying whether the sample is a control fluid or a test fluid. By reference value is meant one value or a series of values, typically corresponding to various time points. The reference value may be a reflectance value or a current value, depending on whether the particular test is a colorimetric or electrochemical test, as will be described in greater detail below.

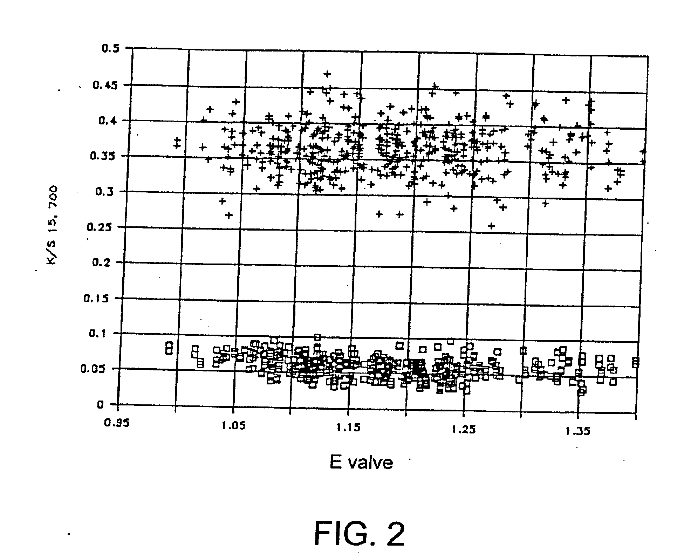

Where the reference value is a reflectance value, i.e., the test is a colorimetric test, the reference value may be a reflectance signal which is any representation or value relating to the quantity of light reflected by the sample over time for one or more wavelengths. For example, the reference reflectance value for a colorimetric assay may include a ratio corresponding to the reflectance of light, where such a ratio may relate to the color intensity of the light reflected by the sample at one or more wavelengths during a period of time, typically observed in about 10-20 seconds from sample introduction to the meter and usually in about 12-18 seconds from sample introduction to the meter. Any suitable ratio may be used, such as a K/S ratio, as is known in the art, where K is the light absorption coefficient in solid phase and S is the light scattering coefficient (see for example U.S. Patent No. 5.049,487, the disclosure of which is incorporated by reference), usually at a wavelength of about 450 to 750 nm, usually about 650 to 720 nm and more usually about 700 nm. Accordingly, the meter is capable of comparing the one or more reference reflectance values to one or more signals generated by the sample at a specific time point or over a period of time, where such period of time is less than about 20 seconds and usually about 12-18 seconds, as described above. A representative evaluation is shown by Figure 2, which shows the plot of K/S values at 15 seconds from the time of sample introduction into the meter at a wavelength of 700 nm against the E coefficient value of the meter for a control fluid and a test fluid. Thus, a K/S value may be set as the reference value such that if the K/S of the sample is greater than the reference K/S value, the sample is a test fluid and if the K/S value of the sample is less than the reference K/S value, the sample is a control fluid. The meter is thus capable of comparing K/S values from the sample to a predetermined reference, i.e., known K/S value, and identifying the sample as a control or a test fluid based upon one or more such comparisons.

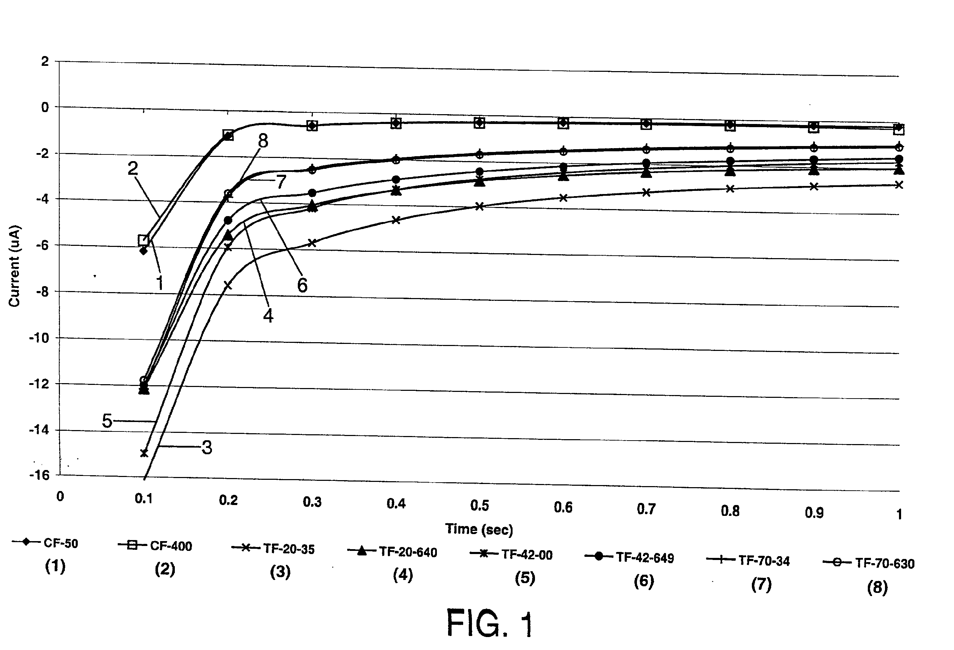

Where the reference value is a current value, i.e., the test is an electrochemical test, the reference value may be one that is related to the magnitude of a signal or to the rate of change of one or more signals generated by an electrochemical reaction, usually observed over a period of time. Typically, such a current signal is one that is observed in less than about 1 second from the time the sample is introduced to the meter and usually observed in less than about 0.25 to 0.75 seconds from sample introduction. The reference current value may be a value such as a numerical value or the like, where such a value relates to the magnitude of current at a particular time point or over a period of time, i.e., the change in the current magnitude over time. Typically, the rate of change in the current of a sample over time will exhibit a substantially constant, ascending or positive slope for at least a short period of time, as described above. A representative graph showing current against time of control fluids and test fluids is shown in Figure 1. Thus, a value relating to an absolute current magnitude or a series of current magnitudes may be set as the reference value(s) such that if the absolute current signal generated from the sample is greater than the reference current value the sample is a test fluid and if the absolute current signal generated from the sample is less than the reference current value the sample is a control fluid. Alternatively, a value relating to the rate of change in current over time may be used as the reference value. For example, if the rate of change in current over time, i.e., the slope of the line relating to the change in current during the period of time of less than about 1 second or usually less than about 0.25-0.75 seconds, generated by the sample is greater than the reference slope value the sample is a control fluid and if the rate of change in current, i.e., slope, is less than the reference slope value the sample is a test fluid.

As described above, the meter also includes a memory element, which can be a digital integrated circuit which stores data and the microprocessor operating program. In many embodiments, the memory element stores a plurality of analyte concentrations. In accordance with the subject invention, the memory element may also be capable of storing a plurality of analyte concentrations of both control fluid and test fluid such that the control and test concentrations remain distinguished from each other in the memory element of the computer. Advantageously, the meter may also be programmed to exclude certain analyte concentration values from its memory while storing others, where such excluding and storing may be accomplished manually or automatically, but typically it will be accomplished automatically. For example, the meter may be capable of automatically excluding analyte concentration values of control fluid while storing analyte concentration values of test fluid.

In many embodiments, the meter also has a computing means, *e.g*., an algorithm, which is capable of averaging a plurality of stored analyte concentration levels stored in its memory, usually without the inclusion of stored analyte concentration levels from different sample types, *e.g.,* averaging stored test fluid data without the inclusion of control fluid data.

### KITS

Also provided by the subject invention are kits for use in practicing the subject methods. The kits of the subject invention include at least one subject device, as described above. The subject kits may also include an element for obtaining physiological sample. For example, where the physiological sample is blood, the subject kits may include an element for obtaining a blood sample, such as a lance for sticking the finger, a lance actuating element, and the like. In addition, the subject kits may include a control fluid as described above, *e.g.,* a glucose control fluid. Certain kits may include one or more test strips. Finally, the kits may further include instructions for using the subject devices for determining the concentration of an analyte in a sample. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette, etc.

### EXPERIMENTAL

The following examples are offered by way of illustration and not by way of limitation.

### I. Electrochemical Analyte Concentration Determination Example

### Preparation of Control Fluid

The control fluid was prepared using the following components and respective quantities.

| Component | Function | Quantity |
|---|---|---|
| Citraconic acid | Buffer | 0.0833 g |
| Dipotassium citraconate | Buffer | 1.931 g |
| Methyl Paraben | Preservative | 0.050 % w/w |
| Germal II | Preservative | 0.400 % w/w |
| Dextran T-500 | Viscosity Modifier | 3.000 % w/w |
| Pluronic 25R2 | Wicking Agent | 0.050 % w/w |
| 1-[(6-methoxy-4-sulfo-m-tolyl)azo]-2-naphthol-6-sulfonic acid disodium salt | Color | 0.100 % w/w |
| D-Glucose | Analyte | 50, 120, or 400 mg |
| Deionized Water | Solvent | 100 g |

First citraconic buffer pH 6.5 ± 0.1 was prepared by dissolving required quantities of acid and salt in deionized water. Next, Methyl Paraben was added and the solution was stirred until the preservative was fully dissolved. Subsequently Dextran T-500, Germal II, Pluronic 25R2 and 1-[(6-methoxy-4-sulfo-m-tolyl)azo]-2-naphthol-6-sulfonic acid disodium salt were added sequentially, following complete dissolution of the previously added chemical. At this point, the pH of the control fluid was verified, followed by addition of the requisite quantity of glucose to obtain a low, normal or high glucose level of control fluid. After the glucose was dissolved completely, the control fluid was left at room temperature overnight. Finally, the glucose concentration was verified using a Model 2700 Select Biochemistry Analyzer manufactured by Yellow Springs Instrument Co., Inc.

### B. Testing

An electrochemical strip, constructed as described above, was inserted into an electrochemical meter and a sample (about 2 µL, or less) of test fluid (*i.e*., whole blood), or control fluid was deposited on the test strip. The meter then measured the current at specified time intervals, as described above, and compared the current magnitude to values preprogrammed into the meter which are characteristic for a particular sample type. The meter then flagged the sample accordingly.

### C. Results

Figure 1 shows a "current response" graph in which the output, *i.e.,* current, from the meter is plotted as a function of time for the control fluid samples, identified as CF, having predetermined glucose concentration levels of 50 mg/dL and 400 mg/dL, and blood samples, identified as TF having target glucose concentrations of 0, 40, and 600 mg/dL. In the case of the control fluid, whether it had a low (50 mg/dL) or high (400 mg/dL) concentration of glucose, the magnitude of the initial current was very small (negative 1 - 2 µA). When test fluid *(i.e.,* whole blood) was applied to the strip, the meter initially detected current values with magnitudes significantly greater than those generated by the control fluid. In Figure 1, the control fluid (CF) is followed by the respective glucose concentration level (sample nos. 1-2) and blood is denoted as TF (sample nos. 3-8) and is followed by the respective % hematocrit and glucose concentration in mg/dL *(e.g.,* sample TF-70-34 refers to whole blood, with 70 % hematocrit and 34 mg/dL of glucose, etc.). The results demonstrated that differentiation between control and test fluids is achieved using test fluids having wide ranges of glucose concentrations and wide levels of red blood cells.

Figure 1 thus shows that throughout the initial first second after sample introduction to the reagent test strip, the current for the control fluids are significantly lower than the currents obtained from the test or blood fluids. Accordingly, the divergent readings of the control and test fluids enable the sample identification element of the meter to flag or identify a sample as either control or test fluid and process the sample accordingly, as described above.

### II. Colorimetric Analyte Concentration Determination Example

### A. Preparation of Control Fluid

The control fluid was prepared using the following components and respective concentrations.

| Component | Function | Final concentration (% w/w) |
|---|---|---|
| Polyvinyl acetate | Polymer, sample penetration and reflectance modifier | 14.3 |
| Copper Phthalocyanine-3,4',4",4"' -tetrasulfonic acid, tetrasodium salt | Dye, reflectance modifier | 0.0075 |
| Aerosil 200 | Dispersant | 0.1 |
| Sodium benzoate | Preservative | 0.2 |
| Disodium EDTA | Preservative | 0.1 |
| Dow B emulsion | Antifoamer | 0.02 |
| D-Glucose | Analyte | 40, 100, 300 mg/dL |
| Distilled Water | Solvent | 85.7 |

Control fluid for a colorimetric assay was prepared by sequentially adding the components listed in the above table to distilled water in an appropriate vessel, while stirring the contents of the vessel. The requisite quantity of glucose was added as the last chemical and the control fluid was then heated to 90 °C in a closed container for 2 hours. Following 24 hours equilibration at room temperature, the glucose concentration was measured by a Model 2700 Select Biochemistry Analyzer manufactured by Yellow Springs Instrument Co., Inc.

### B. Testing

A photometric test strip, constructed as described above, was inserted into an optical meter and at least about 5 µL of the control fluid or whole blood was deposited onto the test strip with a pipette. To differentiate between control and test fluids, the meter measured the reflectance of the strip, at 700 nm, 15 seconds following sample application. The color intensity at specified light wavelengths can be described by K/S, where K is the light absorption coefficient in solid phase and S is the light scattering coefficient (see U.S. Patent No. 5,049,487, the disclosure of which is herein incorporated by reference).

### C. Results

The results show a significant difference in the K/S 15, 700 values between the control and blood fluids.

Figure 2 shows a "reflectance response" graph for the initial 15 seconds after sample introduction to a reagent test strip. In the graph of Fig. 2, output, *i.e.,* K/S at 700 nm reflectance reading, is plotted as a function of meter calibration coefficient E for the control fluid sample having a predetermined glucose concentration level of 40 mg/dL (identified as squares) and a blood sample having a glucose concentrations of 33 - 46 mg/dL and 18 % hematocrit (identified as crosses).

Figure 2 demonstrates that throughout the initial fifteen seconds after sample introduction to the reagent test strip, the control fluid shows significantly lower reflectance signals as compared to the blood sample for a wide range of the E coefficient. Specifically, the mean K/S of the control fluid equals 0.0589 with a standard deviation of 0.0135 while the mean K/S of the blood fluid equals 0.3651 with a standard deviation of 0.0323. Accordingly, the divergent readings of the control and test fluid enable the sample identification element of the meter to flag or identify a sample as either control or test fluid and process the sample accordingly, as described above.

It is evident from the above results and discussions that the above described invention provides a simple and accurate way to identify whether a sample is a control fluid or test fluid in analyte concentration determination assays. The above described invention provides for a number of advantages, including the abilities to store and exclude the analyte concentration value from the memory of a meter, use with simple control solutions and use with both electrochemical and colorimetric assays. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A method for determining the concentration of an analyte in a sample, said method comprising:
a. providing a system comprising:
i) a reagent test strip having a sample receiving region, and
ii) a meter for determining analyte concentration in a sample introduced to said sample receiving region,
b. introducing a sample to said receiving region, wherein said sample is selected from the group consisting of a control fluid and a test fluid, with the proviso that said control fluid is free of a mediator dissolution slowing component and an oxidizing agent when said analyte concentration is determined using electrochemical methods, and
c. determining the concentration of analyte in said sample with said meter, wherein said method further comprises determining whether said sample is a control fluid or a test fluid to obtain sample identification information .

2. The method according to claim 1, wherein said sample is determined to be a control fluid or a test fluid in less than about 20 seconds from the time of sample introduction.

3. The method according to claim 1, further comprising the step of storing said sample identification information in a memory element of said meter.

4. The method according to claim 1, further comprising the step of excluding said sample identification information from a memory element of said meter.

5. The method according to claim 1, wherein said meter measures a signal produced by said control fluid and said test fluids, and said measured signal produced by said control fluid is less than said measured signal produced by said test fluid.

6. A meter for determining the concentration of an analyte in a sample, wherein said meter comprises a sample identification element for identifying whether a sample is a test fluid or a control fluid, wherein said sample identification element comprises at least one reference value selected from the group consisting of a reflectance value and a current value and said current value is one that is observed in less than about 1 second from the time said sample is introduced to said meter.

7. The meter according to claim 6, wherein said meter further comprises a memory element capable of storing a plurality of analyte concentrations.

8. The meter according to claim 7, wherein said memory element is capable of storing said plurality of analyte concentrations of said control fluid and said test fluid distinguishably.

9. The meter according to claim 7, wherein said meter is capable of averaging said plurality of stored analyte concentrations stored in said memory element.

10. The meter according to claim 7, wherein said meter is capable of excluding said analyte concentration of said control fluid from said memory element while storing said analyte concentration of said test fluid in said memory element.

11. A system for determining the concentration of an analyte in a sample, said system comprising:
(a) a reagent test strip having a sample receiving region, and
(b) a meter according to claim 6.
